# EUROPEAN PATENT APPLICATION

(11) **EP 1 302 609 A1**
(43) Date of publication of application: **16.04.2003**
(21) Application number: 02380200.2
(22) Date of filing: 24.09.2002
(51) Int. Cl.: E04G 1/26

(54) **Safety handrail for construction work.**

(30) Priority: 27.09.2001 ES 200102379 U
(71) Applicant: Rodriguez Gonzalez, Luis, 33559 Cangas De onis (Asturias) (ES)
(72) Inventor: Rodriguez Gonzalez, Luis, 33559 Cangas De onis (Asturias) (ES)
(74) Representative: Davila Baz, Angel

(57) **Abstract**

Safety handrail for construction work which is constituted by a first part formed by two posts, linked by a series of perpendicular tubular elements to the same and a second part formed by a third post and a series of tubular elements, of smaller diameter than the previous ones, in which they can be fitted telescopically, the post of this second part and one of the posts of the first part having lower extensions to allow the anchoring of the handrail in the corresponding supports. Both the posts and the anchoring supports have means capable of limiting both the telescopic displacement between the two parts, and between the handrail and the supports.

## Description

### OBJECT OF THE INVENTION

The present invention relates to a safety handrail that has been specially designed to surround the floors of buildings under construction, preventing persons participating in the work from falling off accidentally over the edges of the building panels.

The object of the invention is to obtain a secure handrail, structurally light and strong, modular and extendable, both to reduce its bulk and facilitate its manipulation and to adapt its effective length to the specific requirements of each case, said handrail also resulting simply and firmly secured to the building panel.

Although the handrail has been specially conceived for the application mentioned immediately above, it is equally useful as a barrier for closing streets, entrance to properties, etc.

### BACKGROUND OF THE INVENTION

In the preferred ambit of practical application of the invention, that of construction work, the legal regulatory scheme in force requires that, during the performance of the work, the perimeter of each floor be maintained protected with a handrail of certain structural and dimensional properties, which handrail obviously has to be removed at the appropriate time.

One of the solutions in this respect consists in securing struts to the panel by different means and establishing between said struts means of enclosure which on some occasions are materialized in wooden planks, on others in metallic bars, and even in stout ropes, all these systems having a problem which centres on the complexity of installing the handrail in some cases, and in other cases in the difficulty of manipulation, transport and storage thereof and of all of them none responds to the strength and security properties defined by the regulation.

### DESCRIPTION OF THE INVENTION

The handrail which the invention proposes constitutes a structurally simple and functionally effective solution which resolves in a fully satisfactory manner the conventional problem in this field.

To this end and in a more concrete manner the handrail proposed is constructed by means of two vertical and outer posts, which constitute both the anchor base and the support for the rest of the structure, consisting of a series of horizontal bars, preferably six in number, grouped in pairs, with the special particularity that these horizontal bars are telescopic so that starting from some certain dimensions for the same, corresponding to the operative condition of the handrail, they are capable of having their operative length shortened until about half, in order to reduce the bulk of the handrail in the inoperative condition and to facilitate manipulation thereof.

Both the vertical posts and the horizontal bars are tubular in structure and are constituted by means of a steel and PVC combination which confers upon the structure, in parallel with good structural rigidity, a remarkable lightness in weight, which also contributes to facilitating the manipulation of the handrail.

The encircling horizontal bars or of greater diameter are connected by their extremity opposite to the vertical post which participates in the anchor base by means of a third post, which is interrupted at a lower level than the previous ones, and this third post is connected with the post of the other portion of the handrail by means of chains or the like which limit the extent to which the handrail can be extended, impeding the decoupling of the two telescopic sectors thereof.

Complementary to the structure described, the inclusion has been foreseen in the handrail of a pair of supports, intended to be incorporated previously in the concrete constituting the building panel, supports in which can be nested the lower extremity of the posts constituting the anchor base, there being in said supports a hook for a chain which impedes accidental egress of the posts by connecting each one of them with the aforementioned and corresponding support.

The horizontal bars are essentially of PVC, with the incorporation on the internal extremity of those of smaller diameter of an internal steel tube which serves as stiffener thereof, and the vertical posts which serve as anchor base have an external steel tube of length coincident with that of the post itself; sheathed in another of PVC and a second internal tube, also of steel, adequately secured to the first one, which affects approximately the lower half thereof in order to, with a minimum provision of material, endow the post with the appropriate structural rigidity in the two mentioned sectors thereof.

### DESCRIPTION OF THE DRAWINGS

To complete the description that is being made and with the object of assisting in a better understanding of the characteristics of the invention, in accordance with a preferred example of practical embodiment thereof, said description is accompanied, as an integral part thereof, with a set of drawings wherein by way of illustration and not restrictively, the following has been represented:
Figure 1.- It shows a schematic representation in front elevation of a safety handrail for construction work implemented in accordance with the object of the present invention, which appears in the condition of maximum extension.

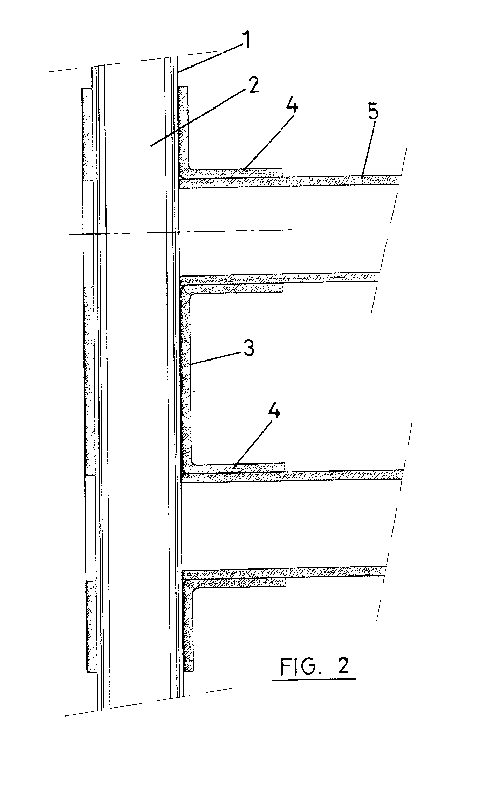

Figure 2.- It shows a partial detail, exploded and sectional of the handrail of the previous figure.

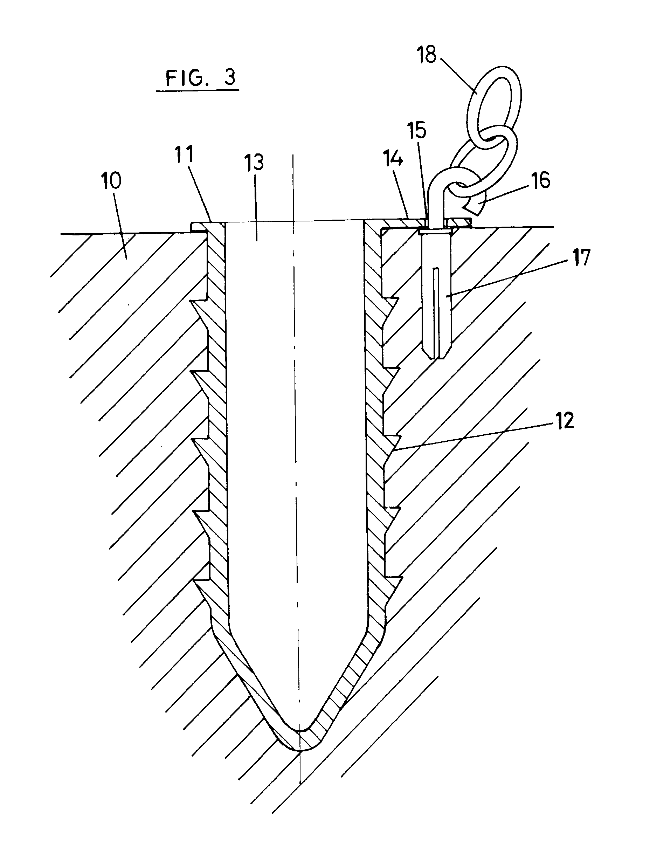

Figure 3.- It shows a view in side elevation and in section of one of the complementary supports of the handrail of figure 1, properly embedded in a building panel.

### PREFERRED EMBODIMENT OF THE INVENTION

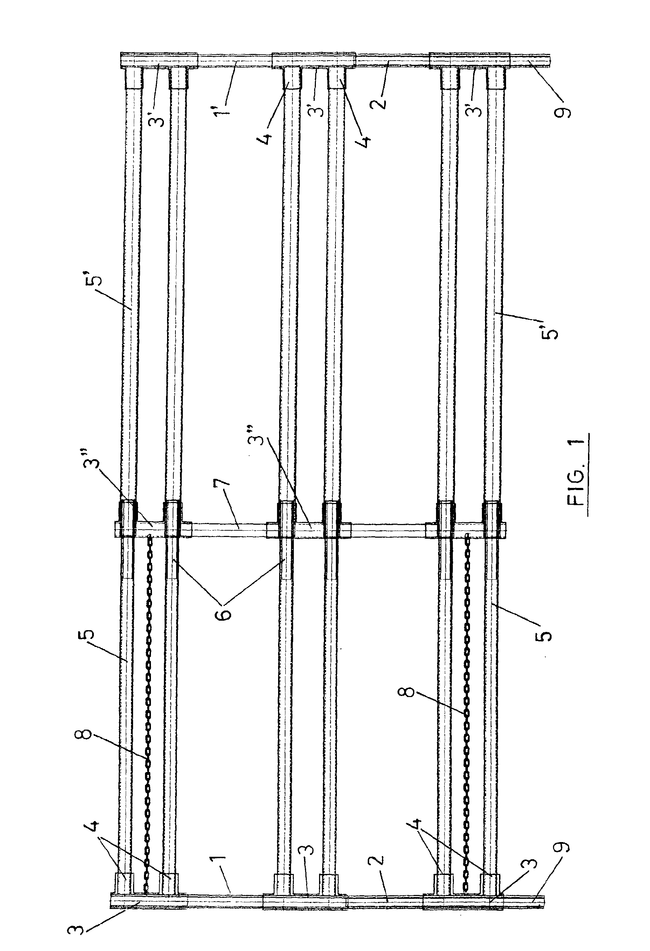

In the light of the figures remarked and especially of figure 1, it can be observed how in the handrail itself two vertical posts (1-1') participate, of steel sheathed in PVC, reinforced in their lower half by means of a second steel tube (2), internal and adequately joined to the external tube (1), there being fitted on each one of these vertical posts (1-1') three robust sleeves (3-3') of PVC, two in end positions and one in an intermediate position, each of which has two necks (4) which project radially from the same in a parallel arrangement, intended to receive inside in an integral manner the ends of individual transversal bars (5-5'), telescopic, so that the length (5) of smaller diameter of these bars, which is reinforced on its internal extremity with an internal steel bush (6), penetrates inside the section (5') of greater diameter, which by its other end is secured to the corresponding vertical post (1') by means of the sleeves (3') equivalent to the previous ones.

The internal end of the lengths (5') of greater diameter of the different bars, these are connected to each other by means of sleeves (3"), also similar to the previous ones, fitted and secured on a vertical and intermediate post (7), so that starting from the condition of maximum extension for the handrail shown in figure 1, which is limited by the presence of a pair of chains (8) fitted between the end sleeves (3-3"), the handrail is capable of being narrowed, adapting to any specific requirement, and of being totally retracted, to a limiting situation wherein its length becomes half of its maximum length, which facilitates its storage, transport and manipulation.

While the vertical and intermediate post (7) is interrupted immediately below the lower outer horizontal bar (5-5'), the outer vertical posts (1-1') have the function of anchor base for which they have lower extensions (9) intended to be secured by nesting in the building panel (10) of the construction job.

In this sense it has been foreseen that with each post (1-1') there is a participating support (11) materialized in a plastic piece, preferably obtained by injection, which is configured in the manner of a plug closed at its internal extremity, with crests (12) on the perimeter for securing to the concrete, said support intended to be constituted beforehand in the latter and which determines a cylindrical lodging (13) dimensionally appropriate to receive therein, snugly, the lower extension (9) of the post (1-1') corresponding.

To assure the immobility between these elements, post (1-1') and support (11), it has been foreseen that said support incorporates at the level of its mouth (14), specifically in a lip being a continuation coplanar with said mouth, a small hole (15) for a hook (16) to pass which is also secured in the concrete (10) with the collaboration of a conventional plug (17) and which constitutes a means of securing for a short chain (18) which connects said hook (16) with any appropriate point of the structure of the handrail itself, like for example with the lower sleeve (3) between its two radial necks (4), to prevent a possible accidental decoupling of the posts (1-1') with respect to the respective supports (11).

In accordance with the structure described and as has already been pointed out, the handrail which the invention proposes is easy to handle, because of its minimal weight and its facility for drastic reduction in its length, which facilitates as well as quick installation and dismantling, transport, packing and storage, said handrail being immune to weather conditions, easy to clean and indefinitely reusable.

## Claims

1. Safety handrail for construction work, which being specially designed to surround each panel of a building in construction, is **characterised in that** it is constituted by means of two vertical posts (1,1') which constitute the anchor base thereof and the support of its structure, in which participate a plurality of telescopic horizontal bars (5,5'), so that said handrail is capable of retracting, from a condition of maximum extension to a condition of maximum retraction, in which its length approaches half that of the first condition.

2. Safety handrail for construction work, according to claim 1, **characterised in that** each horizontal bar is constructed by means of two tubes of different diameter (5,5'), capable of running telescopically one inside the other, the tubes of greater diameter (5') being connected to each other, by their end opposite to the post (1') participant in the anchor base, through a third intermediate post (7) which adequaltely stabilizes said ends, it having been foreseen that between said intermediate post and the post (1) corresponding to the tubes (5) of smaller diameter at least one chain (8) is fitted, acting as constraint for the telescopic extension of the bars (5,5'), which impedes the decoupling of the two tubes (5,5') constituting said bars.

3. Safety handrail for construction work, according to previous claims, **characterised in that** the vertical posts (1,1') which act as anchor base, of steel, with double wall in their lower half, each incorporate three PVC sleeves (3,3'), secured at the mid-point and at the ends thereof and each of them provided with a pair of radial and parallel necks (4), appropriate in form and dimension to receive and to secure inside the corresponding ends of respective horizontal bars (5,5'), which are also of PVC, it having been foreseen that the tubes of smaller diameter (5) which participate in said horizontal bars (5,5') incorporate on their internal extremity, that of coupling to the tubes of greater diameter (5'), an internal tubular reinforcement (6) of steel.

4. Safety handrail for construction work, according to previous claims, **characterised in that** the outer posts (1,1') have lower extensions (9) for the coupling thereof in respective supports (11) previously incorporated in the concrete constituting the building panel (10).

5. Safety handrail for construction work, according to claim 4, **characterised in that** each of the aforementioned supports (11) incorporated in the concrete consists of a kind of plastic plug, of large dimensions, closed on its internal extremity, with perimeter crests (12) for embedding in the concrete (10), defining a lodging (13) appropriate in form and dimension to receive snugly the lower end of said posts (1,1').

6. Safety handrail for construction work, according to claims 4 and 5, **characterised in that** said supports (11) incorporate at the level of their mouth and on the outside thereof a hook (16) for securing a chain (18) which connects each support (11) with any appropriate point of the structure of the handrail itself, in order to prevent the accidental decoupling thereof.
